# EUROPÄISCHE PATENTANMELDUNG

(11) **EP 4 686 461 A1**
(43) Veröffentlichungstag der Anmeldung: **04.02.2026**
(21) Anmeldenummer: 25193477.4
(22) Anmeldetag: 01.08.2025
(51) Int. Cl.: A61F 13/14, A41D 27/12

(54) **TEXTILE HAUTFALTENEINLAGE**

(30) Priorität: 02.08.2024 DE 202024104356 U; 02.08.2024 DE 102024122109
(71) Anmelder: Mondragon, Paola Montano, 01069 Dresden (DE); Neustadt, Lara, 01069 Dresden (DE); Pilk, Tom, 01069 Dresden (DE)
(72) Erfinder: Mondragon, Paola Montano, 01069 Dresden (DE); Neustadt, Lara, 01069 Dresden (DE); Pilk, Tom, 01069 Dresden (DE)
(74) Vertreter: Rauschenbach, Marion

(57) **Zusammenfassung**

Die vorliegende Erfindung bezieht sich auf das technische Gebiet der Textiltechnik und der Medizintechnik und betrifft eine textile Hautfalteneinlage. Die Aufgabe der vorliegenden Erfindung besteht in der Angabe einer Hautfalteneinlage, die einerseits die Aufnahme von Schweiß im Bereich der Hautfalte verbessert, Hautirritationen vermeidet, Hautentzündungen und Bakterienwachstum im Bereich der Hautfalte verhindert und eine einfache Handhabung sowie einen sicheren Tragekomfort ermöglicht. Gelöst wird die Aufgabe mit einer textilen Hautfalteneinlage, Textile Hautfalteneinlage, die mindestens eine erste Lage aus einem hydrophilen Vliesmaterial und eine zweite Lage aus einem hydrophoben Vliesmaterial umfasst, die mindestens in einem Randbereich stoff- und/oder kraftschlüssig verbunden sind, wobei auf der zweiten Lage in einem Teilbereich ein adhäsives Verbindungsmaterial vorhanden ist. Die textile Hautfalteneinlage kann beispielsweise bei der Brust des menschlichen Körpers zusammen mit einem Büstenhalter verwendet werden.

## Beschreibung

Die vorliegende Erfindung bezieht sich auf das technische Gebiet der Textiltechnik und der Medizintechnik und betrifft eine textile Hautfalteneinlage, die beispielsweise als textile Brustfalteneinlage bei der Brust des menschlichen Körpers zusammen mit einem Büstenhalter verwendet werden kann.

In Hautfalten des Körpers auftretender Schweiß und Reibungen können im Bereich der betreffenden Hautpartien zu gesundheitlichen Problemen führen. Eine häufig auftretende Hauterkrankung, insbesondere bei Frauen und übergewichtigen Männern, ist die sogenannte Intertrigo, bei der durch das Reiben von zwei aufeinanderliegenden Hautpartien Reizungen entstehen, die zu Entzündungsreaktionen führen können. Dies ist in der Regel dann der Fall, wenn durch die Wechselwirkung von Schweiß zwischen den betreffenden Hautpartien und den Einfluss von Pilzen oder Bakterien unangenehme Schuppenflechten oder Ekzeme entstehen, die schmerzende Wunden hervorrufen.

Aus dem Stand der Technik sind verschiedene Lösungen bekannt, um in bestimmten Körperpartien des menschlichen Körpers Schweiß aufzufangen und Entzündungen im Bereich der sich berührenden Hautpartien zu vermeiden.

Aus der DE 20 2005 007 355 U1 sind Pads zum Schutz für großflächige Hautbereiche und Problemzonen auf Hautoberflächen zur wirksamen Abhilfe von Hautreizungen und Hautreibungswunden bekannt, bei denen eine Folie vorhanden ist, die einseitig mit einem nicht härtenden farblosen Kleber beschichtet ist und mit einer darüber abziehbaren Schutzfolie versehen ist.

Aus der DE 20 2020 101 217 U1 ist eine Hautfalteneinlage bekannt, bestehend aus einem bikonvex geformten, saugfähigen, flächigen Zellstoff, wobei der flächige Zellstoff eine Faltkante besitzt, die den flächigen Zellstoff in zwei bezüglich der Faltkante spiegelsymmetrische plankonvex geformte Teilbereiche teilt.

Aus der DE 102 02 410 A1 ist ein Absorptionselement mit einer zur Einführung in den submammären Bereich der menschlichen Brust in geeigneter Form bekannt. Das vorgeschlagene Absorptionselement dient der Aufnahme und der Bindung von Körperflüssigkeiten.

Aus der DE 20 2016 002 055 U1 ist eine schweißaufsaugende und druckmindernde Büstenhalter-Einlage bekannt, die zwei halbrunde Ausschnittformen aufweist, die der weiblichen Brust nachempfunden sind und jede Brust einzeln umschließt. Die Einlage ermöglicht, genau dem Verlauf der weiblichen Brust zu folgend und die Büstenhalter-Bügel in ihrer gesamten Länge auf dem Rand der Ausschnittformen zu positionieren, um dadurch den Druck der Büstenhalter-Bügel zu mindern und Hautirritationen vorzubeugen oder zu lindern.

Weiterhin ist aus der DE 20 2004 006 682 U1 eine Einlage bekannt, die im Wesentlichen aus zwei flügelartigen Abschnitten besteht, wobei mindestens ein Abschnitt auf einer ersten Seite als Saugschicht ausgebildet ist. Mindestens ein Abschnitt weist auf der der ersten Seite gegenüberliegenden zweiten Seite ein mit dem Bekleidungsstück zusammenwirkendes und leicht lösbares Verbindungsmittel auf.

Nachteilig bei den bekannten Lösungen ist, dass Hautreizungen, beispielsweise durch hautunverträgliche Klebstoffe, auftreten. Insbesondere kann das Lösen der von bekannten Pads oder Pflastern von der Haut schmerzhaft sein und zu juckenden Allergien führen. Zudem ist nachteilig, dass die Einlagen verrutschen, wodurch Falten entstehen und zusätzliche Reizungen der Haut beim Tragen entstehen. Außerdem ist es nachteilig, dass die Reibung zwischen den Hautpartien nur unzureichend verhindert wird.

Die Aufgabe der vorliegenden Erfindung besteht in der Angabe einer Hautfalteneinlage, die einerseits die Aufnahme von Schweiß im Bereich der Hautfalte verbessert, Hautirritationen vermeidet, Hautentzündungen und Bakterienwachstum im Bereich der Hautfalte verhindert und eine einfache Handhabung sowie einen sicheren Tragekomfort ermöglicht.

Die Aufgabe wird durch die in den Patentansprüchen angegebene Erfindung gelöst. Vorteilhafte Ausgestaltungen sind Gegenstand der Unteransprüche, wobei die Erfindung auch Kombinationen der einzelnen abhängigen Patentansprüche im Sinne einer Und-Verknüpfung einschließt, solange sie sich nicht gegenseitig ausschließen.

Erfindungsgemäß wird die Aufgabe mit einer textilen Hautfalteneinlage gelöst, die die Absorption von Schweiß und Feuchtigkeit im Bereich der Hautfalte verbessert, Hautirritationen vermeidet, Hautentzündungen verhindert und eine einfache Handhabung sowie einen sicheren Tragekomfort ermöglicht.

Gelöst wird die Aufgabe mit einer textilen Hautfalteneinlage, die mindestens eine erste Lage aus einem hydrophilen Vliesmaterial und eine zweite Lage aus einem hydrophoben Vliesmaterial umfasst, die mindestens in einem Randbereich stoff- und/oder kraftschlüssig verbunden sind, wobei auf der zweiten Lage in einem Teilbereich ein adhäsives Verbindungsmaterial vorhanden ist.

Vorteilhafterweise sind mindestens die erste und zweite Lage als Nähgewirk mit parallel zueinander verlaufenden Maschenreihen zu einem textilen Flächengebilde gewirkt, wobei besonders vorteilhaft die Maschen des Nähgewirks auf der zweiten Lage angeordnet sind.

Auch ist es von Vorteil, wenn das Nähgewirk mit einem natürlichen, künstlichen und/oder biologisch abbaubaren Garn ausgebildet ist, wobei besonders vorteilhaft das Garn aus Polypropylen oder Polystyrol ist.

In einer vorteilhaften Ausgestaltung der textilen Hautfalteneinlage kann vorgesehen sein, dass das Nähgewirk mit 10 bis 25 Fäden pro 25 mm ausgebildet ist.

Zudem ist es vorteilhaft, wenn das Vliesmaterial der ersten Lage aus natürlichen, künstlichen und/oder biologisch abbaubaren Fasern und/oder Filamenten besteht.

Weiterhin ist es von Vorteil, wenn die erste Lage aus Cellulose-Regeneratfasern besteht und eine Feinheit von 0,5 dtex bis 15 dtex aufweist.

Vorteilhafterweise weist das Vliesmaterial der zweiten Lage Polypropylen oder Polysterol auf.

In einer vorteilhaften Ausgestaltung der textilen Hautfalteneinlage kann vorgehen sein, dass die erste Lage hautpflegende Zusätze enthält und/oder die zweite Lage eine reibungsmindernde Beschichtung aufweist.

Auch von Vorteil ist es, wenn zwischen der ersten Lage aus einem hydrophilen Vliesmaterial und der zweiten Lage aus einem hydrophoben Vliesmaterial eine Zwischenschicht angeordnet ist.

Von Vorteil ist es weiterhin, wenn die textile Hautfalteneinlage aus einem konkav ausgebildeten Teilbereich und einem konvex ausgebildeten Teilbereich besteht, wobei das adhäsive Verbindungsmaterial im konkav ausgebildeten Teilbereich angeordnet ist. Besonders vorteilhaft ist dabei, wenn eine bogenförmige Faltkante vorhanden ist, die an die Form des konkav ausgebildeten Teilbereiches angepasst ist.

Zudem ist es von Vorteil, wenn die textile Hautfalteneinlage in horizontaler und/oder vertikaler Erstreckung spiegelsymmetrisch kongruent ausgebildet ist.

Vorteilhafterweise kann vorgesehen sein, dass die Maschenreihen des Nähgewirkes quer zur Positionierung der Einlage in der Hautfalte vorgesehen sind.

Auch ist es von Vorteil, wenn die textile Hautfalteneinlage ein Einmalprodukt ist.

Und auch ist es von Vorteil, wenn mindestens die Lagen thermisch und/oder mittels eines Klebstoffes verbunden sind.

Verwendung kann die textile Hautfalteneinlage insbesondere als textile Brustfalteneinlage finden.

Erreicht werden die technischen Vorteile und Verbesserungen mit einer textilen Hautfalteneinlage, die als textiles Nähgewirk ausgebildet sein kann und mindestens eine erste Lage aus einem hydrophilen Vliesmaterial und eine zweite Lage aus einem hydrophoben Vliesmaterial umfasst.

Das vorgeschlagene hydrophile Vliesmaterial zeichnet sich durch ein besonders hohes Absorptionsvermögen aus, wodurch der in der Hautfalte entstehende Schweiß vom Vliesmaterial schnell aufgenommen und das Bakterienwachstum wirkungsvoll unterbunden wird. Zudem werden durch das hydrophile Vliesmaterial ein angenehmer Tragekomfort mit geringer Hautreibung ermöglicht.

Insbesondere für den angenehmen Tragekomfort und die schweißabsorbierenden Eigenschaften hat sich als vorteilhaft herausgestellt, wenn die erste Lage aus dem hydrophilen Vliesmaterial aus Cellulose-Regeneratfasern besteht und eine Feinheit von 0,5 dtex bis 15 dtex aufweist.

Als vorteilhaft hat sich für die erste hydrophile Vliesmateriallage erwiesen, wenn das Vliesmaterial natürliche, künstliche und/oder biologisch abbaubare Fasern und/oder Filamente aufweist. Auch für die zweite hydrophobe Vliesmateriallage kann vorgesehen sein, dass diese Vliesmateriallage aus natürlichen, künstlichen und/oder biologisch abbaubaren Fasern und/oder Filamente besteht, besonders vorteilhaft aus Polypropylen oder Polysterol. Durch den Einsatz von Fasern und/oder Filamente aus Polypropylen oder Polysterol werden die mechanischen Eigenschaften dieser Lage bei tribologischer Beanspruchung verbessert und die Reibung mit dem in Kontakt stehenden Kleidungsstück verringert. Dadurch wird zudem das Verrutschen oder Aufstauen der Hautfalteneinlage zwischen dem Kleidungsstück und der darunter liegenden Haut wirksam vermieden.

Die beiden unterschiedlichen Vlieslagen können vorteilhafterweise durch parallel zueinander verlaufende axiale Maschenreihen zu einem textilen Flächengebilde gewirkt und miteinander verbunden sein. Durch die zueinander parallel verlaufenden axialen Maschenreihen wird eine dauerhafte und sichere Verbindung mindestens der ersten hydrophilen Vlieslage mit der zweiten hydrophoben Vlieslage erreicht und eine hohe Formstabilität der textilen Hautfalteneinlage erzeugt.

Zum Erreichen eines Kosten-Nutzen-Vorteils kann vorteilhafterweise vorgesehen sein, dass das Nähgewirk mit 10 bis 25 Fäden pro 25 mm ausgebildet ist. Eine textile Hautfalteneinlage mit einem Nähgewirk mit 10 bis 25 Fäden pro 25 mm weist eine hohe Formstabilität und sicheren Verbund der ersten hydrophilen Vlieslage und zweiten hydrophoben Vlieslage auf und ist zudem günstig in der Herstellung.

Zum Erreichen einer hohen Längsfestigkeit mit einer hohen Quer-Dehnfestigkeit kann vorgesehen sein, dass die Maschenreihen des Nähgewirkes quer zur Breite der textilen Hautfalteneinlage und damit quer zur Positionierung der Einlage in der Hautfalte angeordnet sind.

In einer anderen vorteilhaften Ausgestaltung der textilen Hautfalteneinlage kann aus Gründen einer kostengünstigen Herstellung vorgesehen sein, dass die erste und zweite Lage des Vliesmaterials zumindest im Randbereich verklebt oder thermisch verschweißt sind.

Zur verbesserten Aufnahme von Schweiß und Feuchtigkeit ist es von Vorteil, wenn das Garn des Nähgewirks aus einem natürlichen, künstlichen und/oder biologisch abbaubaren Material besteht.

In einer vorteilhaften Ausgestaltung der Hautfalteneinlage kann vorgesehen sein, dass die Maschen des Nähgewirks auf der zweiten hydrophoben Vlieslage angeordnet sind. Der technische Vorteil der Anordnung der Maschen auf der zweiten hydrophoben Vlieslage besteht darin, dass durch das mit den erhobenen Maschen erzeugte Oberflächenprofil die Kontaktfläche mit dem Kleidungsstück verringert und zudem eine dämpfende Wirkung erzeugt wird. Dadurch wird die Reibung zwischen der hydrophoben zweiten Vlieslage mit dem Kleidungsstück signifikant reduziert. Außerdem wird durch die Anordnung der Maschen auf der zweiten Vlieslage eine dämpfende Wirkung erreicht, die den Tragekomfort der textilen Hautfalteneinlage zusätzlich verbessert.

Um das tribologische System weiter zu verbessern, kann vorteilhafterweise vorgesehen sein, dass bei der textilen Hautfalteneinlage das Nähgewirk mit einem reibungsmindernden Garn ausgebildet ist. Es hat sich als besonders vorteilhaft herausgestellt, wenn Garne aus einem natürlichen, künstlichen und/oder biologisch abbaubaren Material eingesetzt werden, wobei besonders vorteilhaft ein Garn aus Polypropylen oder Polystyrol ist. Nähgewirke mit einem Garn aus Polypropylen oder Polystyrol sind günstig in der Herstellung und wirken zusätzlich reibungsmindernd.

Natürliche und biologisch abbaubare Garne erfüllen insbesondere beim Einsatz der textilen Hautfalteneinlage als Einmalprodukt die gestiegenen Anforderungen an besonders umweltfreundliche und ökologische Produkte.

Die erfindungsgemäße textile Hautfalteneinlage wird über das adhäsive Verbindungsmaterial, dass auf der zweiten hydrophoben Lage mindestens in einem Teilbereich angeordnet ist, mit einem beliebigen Kleidungsstück kontaktiert und an diesem befestigt.

Insbesondere bei der Verwendung der textilen Hautfalteneinlage als textile Brustfalteneinlage kann vorgesehen sein, dass die textile Hautfalteneinlage mit einem konkav ausgebildeten Teilbereich der zweiten hydrophoben Vlieslage innerhalb eines Büstenhalters positioniert und an diesem innenseitig befestigt wird. Der wesentliche Vorteil des adhäsiven Verbindungsmaterials im Teilbereich der zweiten hydrophoben Vlieslage besteht darin, dass das adhäsive Verbindungsmaterial mit dem inneren Stoff des Büstenhalters verbunden wird, sodass die Kontaktierung des adhäsiven Verbindungsmaterials mit der Haut verhindert wird. Dadurch können Hautirritationen aufgrund möglicher Hautunverträglichkeiten mit dem adhäsiven Verbindungsmaterial vermieden werden. Zudem besteht ein weiterer technischer Vorteil darin, dass das Entfernen einer benutzten Hautfalteneinlage schmerzfrei ohne Hautirritationen und in einfacher Weise aus dem Inneren des Büstenhalters ohne Kontakt mit der Haut im Bereich der Brustfalte erfolgt.

Die textile Hautfalteneinlage kann für den Fall, dass sie konkret als textile Brustfalteneinlage verwendet wird, eine an die Brust des menschlichen Körpers angepasste Form aufweisen, um einerseits eine sichere Positionierung im Büstenhalter und andererseits eine vollflächig aufliegende Hautfalteneinlage zwischen der Haut des Oberkörpers, der Brustfalte und der Brust zu erreichen. Hierfür kann vorteilhafterweise die textile Hautfalteneinlage eine halbmondförmige Form mit einem konkav ausgebildeten Teilbereich und einem konvex ausgebildeten Teilbereich aufweisen, wobei das adhäsive Verbindungsmaterial im konkav ausgebildeten Teilbereich angeordnet ist. Damit wird erreicht, dass der konkav ausgebildete Bereich sich faltenfrei an die Form der Brust anpasst, während der konvex ausgebildete Teilbereich faltenfrei zwischen der Brust und dem Oberkörper im Bereich der Brustfalte angeordnet ist.

Ein weiterer technischer Vorteil der erfindungsgemäßen textilen Hautfalteneinlage besteht darin, dass bei Ausbildung der vorgenannten speziellen Form die textile Hautfalteneinlage in horizontaler und/oder vertikaler Erstreckung spiegelsymmetrisch kongruent ist, sodass die Brustfalteneinlage im Bereich der Brustfalte passgenau eingesetzt werden kann. Die textile Hautfalteneinlage steht dabei mit dem bogenförmigen Bügel eines Büstenhalters in Kontakt, wodurch die Haut geschützt und der Druck des Bügels auf die Haut reduziert werden. Insbesondere an dieser Hautpartie wird der Schweiß absorbiert und zusätzlich die Reibung des Bügels des Büstenhalters in der Brustfalte verringert. Dies wird dadurch erreicht, dass die textile Hautfalteneinlage im Inneren des Büstenhalters mit der hydrophilen Vlieslage über das adhäsive Verbindungsmaterial befestigt wird, sich aus dem Inneren des Büstenhalters erstreckt und zwischen der Brust und der Haut des Oberkörpers schützend liegt.

In einer weiteren vorteilhaften Ausgestaltung kann vorgesehen sein, dass eine Faltkante vorhanden ist, die an die Form des konkav ausgebildeten Teilbereiches angepasst ist. Eine derartige Faltkante ist beispielsweise an die Form des Bügels des Büstenhalters angepasst und führt zu einer besseren Formstabilität der textilen Hautfalteneinlage und erleichtert die genaue Positionierung innerhalb des Kleidungsstückes.

Um während des Tragens der textilen Hautfalteneinlage gleichzeitig einen Schutz und/oder Pflege von möglicherweise bereits entzündeten Hautpartien zu ermöglichen, kann vorteilhafterweise vorgesehen sein, dass die erste hydrophile Vlieslage hautpflegende Zusätze enthält.

Auch ist vorstellbar, dass zur weiteren Minderung der Reibung die zweite Lage eine reibungsmindernde Beschichtung aufweist.

In einer vorteilhaften Ausgestaltung der textilen Hautfalteneinlage kann vorgesehen sein, dass zwischen der ersten hydrophilen Vlieslage und der zweiten hydrophoben Vlieslage eine Zwischenschicht angeordnet ist, die mit dem Nähgewirk mindestens teilweise verbunden ist. Die Zwischenschicht ermöglicht eine verbesserte und größere Aufnahme und Speicherung von Schweiß und Flüssigkeit, wodurch insbesondere die Bildung und das Wachstum von Bakterien und Pilzen in der betreffenden Hautpartie wirkungsvoll zumindest verringert wird, da der aufgenommene Schweiß und die Feuchtigkeit von der hydrophilen Vlieslage in die Zwischenschicht weitergeleitet wird. Die Zwischenschicht kann aus einem natürlichen, künstlichen und/oder biologisch abbaubaren und saugfähigem Material bestehen.

Auch ist vorstellbar, dass die textile Falteneinlage mehrere Lagen des hydrophoben und/oder hydrophilen Vliesmaterials oder auch weitere Zwischenschichten aufweist, wodurch eine verbesserte Feuchtigkeitsaufnahme bei verminderter Reibung ermöglicht wird.

Zusammenfassend bestehen mit der erfindungsgemäßen textilen Hautfalteneinlage die technischen Vorteile und Wirkungen gegenüber dem Stand der Technik darin, dass
- ein verbesserter Tragekomfort ermöglicht wird,
- die zu Entzündungen führende Reibung zwischen der Hautpartien untereinander und auch dem Kleidungsstück verringert wird,
- eine verbesserte Aufnahme von Schweiß im Bereich der Hautfalte ermöglicht wird,
- Hautreizungen verhindert werden,
- die Handhabbarkeit durch einfaches Anlegen und Entfernen erleichtert wird und
- eine hohe Formstabilität durch das nähgewirkte Flächengebilde aus der hydrophilen und hydrophoben Vlieslage erreicht wird.

Nachfolgend wird die Erfindung anhand eines Ausführungsbeispiels näher erläutert. Die dazugehörigen Figuren zeigen
- Figur 1: eine schematische Draufsicht auf eine Ausführungsform einer textilen Hautfalteneinlage als Brustfalteneinlage, und
- Figur 2: einen schematischen Querschnitt des Aufbaus einer textilen Hautfalteneinlage als Brustfalteneinlage.

### Ausführungsbeispiel

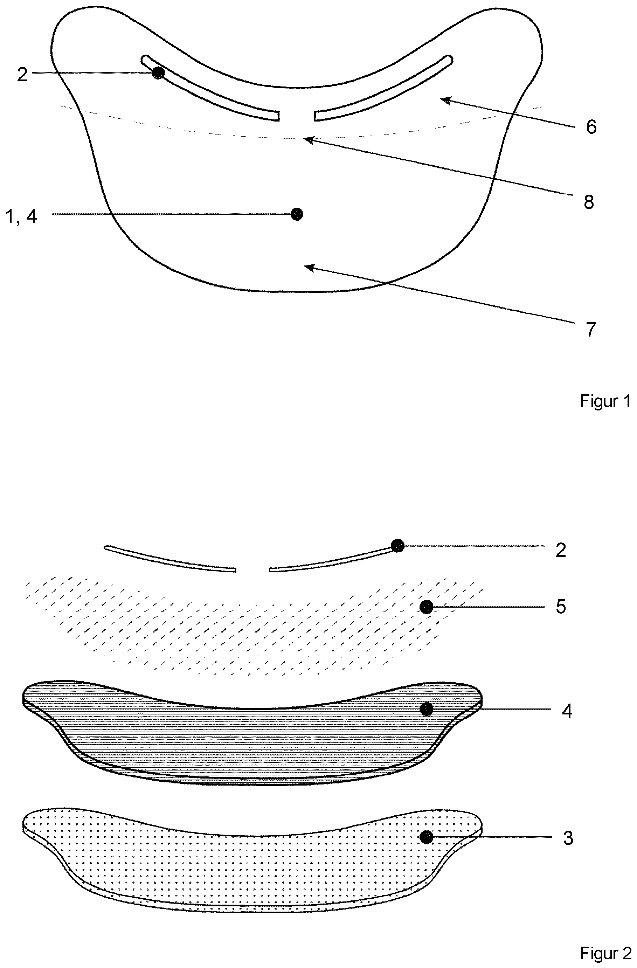

Figur 1 und 2 zeigen eine textile Brustfalteneinlage 1 mit einer halbmondförmigen Form aus einem konkav ausgebildeten Teilbereich 6 und einem konvex ausgebildeten Teilbereich 7. Die Brustfalteneinlage weist eine Breite von 250 mm und eine Höhe von 150 mm auf. Die erste Lage 3 ist aus einem hydrophilen Vliesmaterial, das aus Lyocell ist. Die zweite Lage 4 ist aus einem hydrophoben Vliesmaterial und ist aus Polypropylen. Die erste und zweite Vlieslage 3,4 sind durch parallel zueinander verlaufende Maschenreihen 5 zu einem textilen Flächengebilde als Nähgewirk gewirkt, wobei quer zur Breite 14 Maschenreihen je 25 mm axial gewirkt sind. Das Garn des Nähgewirks 5 ist aus Polypropylen. Die zweite hydrophobe Vlieslage 4 weist in einem Randbereich des konkav ausgebildeten Teilbereiches 6 zwei Streifen eines wiederlösbaren Klebstoffes auf, die mit einer Schutzfolie überzogen sind.

Zur einmaligen Verwendung der textilen Brustfalteneinlage 1 werden die beiden Streifen der Schutzfolie vom wiederlösbaren Klebstoff 2 entfernt und der hydrophobe und konkav ausgebildete Teilbereich 6 im Inneren des Büstenhalters mit dessen Stoff verbunden. Die Positionierung der Brustfalteneinlage 1 erfolgt dabei derart, dass die bogenförmige Faltkante 8 im Wesentlichen parallel mit dem Bügel des Büstenhalters angeordnet wird. Anschließend wird der Büstenhalter an die Brust angelegt, wodurch sich der konvex ausgebildete Teilbereich 7 außerhalb des Büstenhalters im Bereich der Brustfalte und zwischen der Außenseite des Büstenhalters und der Haut des Oberkörpers befindet. Die textile Brustfalteneinlage 1 ist vollständig von der Brust verdeckt, von außen nicht sichtbar und kann auftretenden Schweiß, insbesondere im Bereich der Brustfalte, absorbieren. Die direkte Reibung zwischen Brust und der Haut unterhalb der Brust wie auch durch den Bügel des Büstenhalters in der Brustfalte wird wirkungsvoll verhindert. Zur Entsorgung der textilen Brustfalteneinlage 1 wird der Büstenhalter abgenommen, die verbrauchte Einlage in einfacher Weise und rückstandsfrei vom Stoff des Büstenhalters gelöst und umweltfreundlich entsorgt.

### Bezugszeichenliste

- 1: Textile Hautfalteneinlage
- 2: Adhäsives Verbindungsmaterial
- 3: erste Lage eines hydrophilen Vliesmaterials
- 4: zweite Lage eines hydrophoben Vliesmaterials
- 5: Nähgewirk
- 6: konkav ausgebildeten Teilbereich
- 7: konvex ausgebildeter Teilbereich
- 8: Faltkante

## Patentansprüche

1. Textile Hautfalteneinlage (1), die mindestens eine erste Lage aus einem hydrophilen Vliesmaterial (3) und eine zweite Lage aus einem hydrophoben Vliesmaterial (4) umfasst, die mindestens in einem Randbereich stoff- und/oder kraftschlüssig verbunden sind, wobei auf der zweiten Lage in einem Teilbereich ein adhäsives Verbindungsmaterial (2) vorhanden ist.

2. Textile Hautfalteneinlage nach Anspruch 1, bei der mindestens die erste und zweite Lage (3, 4) als Nähgewirk (5) mit parallel zueinander verlaufenden Maschenreihen zu einem textilen Flächengebilde gewirkt sind und/oder die Maschen des Nähgewirks (5) auf der zweiten Lage (4) angeordnet sind.

3. Textile Hautfalteneinlage nach Anspruch 1, bei der das Nähgewirk (5) mit einem natürlichen, künstlichen und/oder biologisch abbaubaren Garn ausgebildet ist, wobei besonders vorteilhaft das Garn aus Polypropylen oder Polystyrol ist.

4. Textile Hautfalteneinlage nach Anspruch 1, bei der das Nähgewirk (5) mit 10 bis 25 Fäden pro 25 mm ausgebildet ist.

5. Textile Hautfalteneinlage nach Anspruch 1, bei der das Vliesmaterial der ersten Lage (3) aus natürlichen, künstlichen und/oder biologisch abbaubaren Fasern und/oder Filamenten besteht.

6. Textile Hautfalteneinlage nach Anspruch 1, bei der die erste Lage aus Cellulose-Regeneratfasern besteht und eine Feinheit von 0,5 dtex bis 15 dtex aufweist.

7. Textile Hautfalteneinlage nach Anspruch 1, bei der das Vliesmaterial der zweiten Lage (4) Polypropylen oder Polysterol aufweist.

8. Textile Hautfalteneinlage nach Anspruch 1, bei der die erste Lage (3) hautpflegende Zusätze enthält und/oder die zweite Lage (4) eine reibungsmindernde Beschichtung aufweist.

9. Textile Hautfalteneinlage nach Anspruch 1, bei der zwischen der ersten Lage (3) aus einem hydrophilen Vliesmaterial und der zweiten Lage (4) aus einem hydrophoben Vliesmaterial eine Zwischenschicht angeordnet ist.

10. Textile Hautfalteneinlage nach Anspruch 1, die aus einem konkav ausgebildeten Teilbereich (6) und einem konvex ausgebildeten Teilbereich (7) besteht, wobei das adhäsive Verbindungsmaterial (2) im konkav ausgebildeten Teilbereich (6) angeordnet ist und/oder eine bogenförmige Faltkante (8) vorhanden ist, die an die Form des konkav ausgebildeten Teilbereiches (6) angepasst ist.

11. Textile Hautfalteneinlage nach Anspruch 10, die in horizontaler und/oder vertikaler Erstreckung spiegelsymmetrisch kongruent ausgebildet ist.

12. Textile Hautfalteneinlage nach Anspruch 1, bei der die Maschenreihen des Nähgewirkes (5) quer zur Positionierung der Einlage in der Hautfalte vorgesehen sind.

13. Textile Hautfalteneinlage nach Anspruch 1, die ein Einmalprodukt ist.

14. Textile Hautfalteneinlage nach Anspruch 1, bei dem mindestens die Lagen (3, 4) thermisch und/oder mittels eines Klebstoffes verbunden sind.

15. Verwendung der textilen Hautfalteneinlage nach mindestens einem der vorhergehenden Ansprüche als textile Brustfalteneinlage.
